(19) Europäisches Patentamt
European Patent Office
Office européen des brevets

(11) Publication number: **0 184 331**
**A2**

# (12) EUROPEAN PATENT APPLICATION

(21) Application number: 85308043.0

(22) Date of filing: 05.11.85

(51) Int. Cl.[4]: C 12 N 7/06
C 07 D 493/04

(30) Priority: 05.11.84 US 668442

(43) Date of publication of application: 11.06.86 Bulletin 86/24

(84) Designated Contracting States: BE DE FR GB IT LU NL

(71) Applicant: E.I. DU PONT DE NEMOURS AND COMPANY
1007 Market Street
Wilmington Delaware 19898(US)

(72) Inventor: Hanson, Carl Veith
1427 Edith Street
Berkeley California 94703(US)

(72) Inventor: Lambert, Stephen Burton
P.O. Box 723
Groton Massachusetts 01450(US)

(72) Inventor: Richards, James Carlton
290 Ardmore Road
Des Plaines Illinois 60016(US)

(74) Representative: Myerscough, Philip Boyd et al,
J.A.Kemp & Co. 14, South Square Gray's Inn
London, WC1R 5EU(GB)

## (54) Psoralen inactivation of human T-cell leukemia virus III (HTLV III).

(57) A chemical method for the rapid inactivation of human tumor leukemia virus III (HTLV III) without substantial loss of antigenicity using psoralen and selected derivatives thereof and exposure to light of defined wavelength.

./...

Croydon Printing Company Ltd

FIG.1

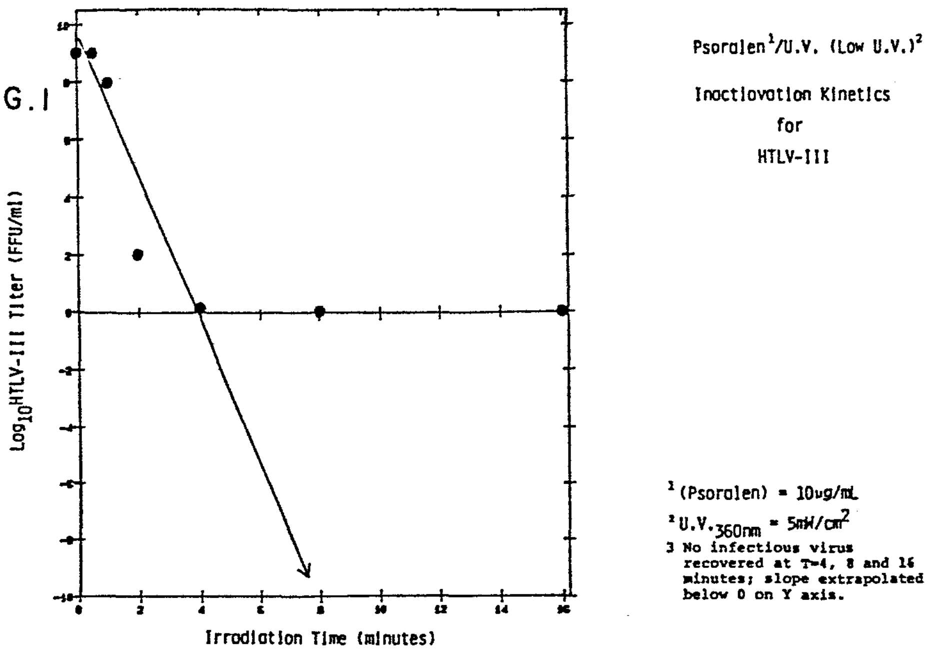

Psoralen[1]/U.V. (Low U.V.)[2]
Inactiovation Kinetics
for
HTLV-III
Log10HTLV-III Titer (FFU/ml)
Irradiation Time (minutes)
[1] (Psoralen) = 10μg/mL
[2] U.V.360nm = 5mW/cm2
3 No infectious virus recovered at T=4, 8 and 16 minutes; slope extrapolated below 0 on Y axis.


FIG.2

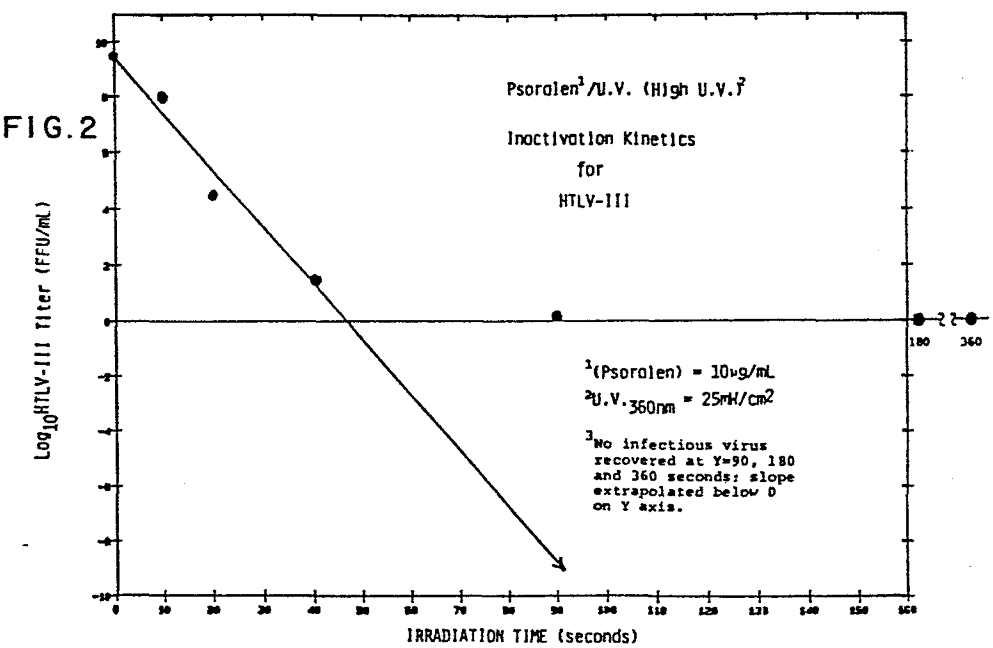

Psoralen[1]/U.V. (High U.V.)[2]
Inactivation Kinetics
for
HTLV-III
Log10HTLV-III Titer (FFU/mL)
IRRADIATION TIME (seconds)
180
360
[1](Psoralen) = 10μg/mL
[2]U.V.360nm = 25mW/cm2
[3]No infectious virus recovered at Y=90, 180 and 360 seconds; slope extrapolated below 0 on Y axis.

TITLE

# PSORALEN INACTIVATION OF HUMAN TUMOR LEUKEMIA VIRUS III

## TECHNICAL FIELD

The present invention relates to a method for the rapid inactivation of Human Tumor Leukemia Virus (HTLV) III without substantial loss of antigenicity.

## BACKGROUND

The use of psoralen and various derivatives thereof to inactivate viruses is well known in both the patent and nonpatent literature. The term "inactivation" as used herein denotes the modification of a virus to eliminate its infectivity.

U.S. Patent 4,124,598 issued November 7, 1978 to Hearst et al. discloses various psoralen derivatives which are useful for inactivating viruses. Psoralen and its useful derivatives have the structure indicated by the formula below:

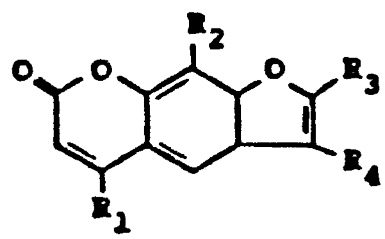

| Name | $R_1$ | $R_2$ | $R_3$ | $R_4$ |
|---|---|---|---|---|
| Psoralen | H | H | H | H |
| TMP (4,5',8-trimethylpsoralen) | $CH_3$ | $CH_3$ | $CH_3$ | H |
| AMT (4'-aminomethyl 4,5',8-trimethylpsoralen) | $CH_3$ | $CH_3$ | $CH_3$ | $-CH_2NH_2$ |
| HMT (4'-hydroxymethyl 4,5',8-trimethylpsoralen) | $CH_3$ | $CH_3$ | $CH_3$ | $-CH_2OH$ |
| MMT (4'-methoxymethyl 4,5',8-trimethylpsoralen) | $CH_3$ | $CH_3$ | $CH_3$ | $-CH_2OCH_3$ |

Musajo et al. disclose five psoralen derivatives (and three other related furocoumarins) which were shown to photoreact well with DNA, but poorly with RNA. Three DNA viruses (pseudorabies, canine hepatitis virus and fowl pox virus) were photoinactivated in the presence of nonderivatized psoralen, while three RNA viruses (NewCastle disease virus, foot-and-mouth disease virus and Teschen disease virus were resistant to an even longer period of psoralen photoinactivation. The RNA viruses resisted up to 120 minutes of irradiation by a quartz lamp (with 366 nm filter) at a distance of 25 cm (intensity not reported) in the presence of 18.7 µg/ml of psoralen. [L. Musajo, G. Rhodighiero, G. Colombo, V. Torlone & F. Dall'Acqua, "Photosensitizing furocoumarins: interaction with DNA and photoinactivation of DNA containing viruses", Experientia 21: 22-24 (1964).]

Bordin et al. obtained leukemic cells by infection of mice with Graffi's leukemia virus. Irradiation of these cells for 30 minutes at 11.5 $mW/cm^2$ of ultraviolet light (365 mm) in the presence of 10 µg/ml of nonderivatized psoralen, 8-methylpsoralen, or 4,4',8-trimethylpsoralen killed the cells (i.e., they were unable to replicate or induce leukemia in adult mice), but "the properties of the [intracellular] Graffi virus remained unchanged and it could infect cells and transform them into leukaemic and virus-producing cells." [F. Bordin and F. Baccichetti, "The furocoumarin photosensitizing effect on the virus-producing Graffi leukemia cells", Z. Naturforsch. 29c: 630-632 (1974).]

Hearst et al. were the first to publish successful photoinactivation of an RNA virus by psoralen derivatives. Vesicular stomatitis virus (VSV) was photoinactived with HMT, MMT or AMT, all of

which were a thousand times more effective than TMP. Two DNA viruses (herpes simplex and vaccinia) were a thousand times more sensitive to photoinactivation by the new psoralen derivatives in comparison to the RNA virus (VSV). Irradiation was performed at 0.7 $mW/cm^2$ of long-wave UV light. At 20-30 µg/ml, HMT, MMT and AMT were "equivalent with respect to light dose response [in the inactivation of VSV]". At 2-3 µg/ml AMT was superior to HMT and MMT in the photoinactivation of VSV. [J. R. Hearst & L. Thiry, "The photoinactivation of an RNA animal virus, vesicular stomatitis virus, with the aid of newly synthesized psoralen derivatives," Nucleic Acids Research 4:1339-1347 (1977).]

In an unpublished report, Hanson showed that Harvey sarcoma virus (a murine retrovirus) required 15 minutes irradiation at 3 $mW/cm^2$ in the presence of 10 µg/ml AMT for a $10^3$-fold inactivation (assayed by counting the foci of virus-transformed indicator cells). Moloney sarcoma virus, another murine retro-virus, underwent a $10^4$-fold inactivation under these same conditions. [C. V. Hanson, "Photochemical Inactivation of Oncogneic Viruses and Tumor Cells", FINAL REPORT (December 15, 1977) for Special Grant No. 864; California Division of the American Cancer Society.]

Hanson et al. showed that on a molar basis, TMP was more effective (within its solubility range of approximately 0-1 µg/ml) than AMT in the photoinactivation of Western equine encephalitis virus (WEE, an RNA virus) and herpes simplex virus (a DNA virus). The greater solubility of AMT, however, resulted in superior inactivation rates by AMT at higher psoralen derivative concentrations. For example, WEE virus underwent a $10^8$-fold inactivation in approximately 5

minutes of irradiation at 1.9 $mW/cm^2$ in the presence of 10 µg/ml AMT. [C. V. Hanson, J. L. Riggs and E. H. Lennette, "Photochemical inactivation of DNA and RNA viruses by psoralen derivatives", J. General Virology 40: 345-358 (1978).]

Nakashima et al. demonstrated the photoinactivation of reovirus (a double-stranded RNA virus) with irradiation at 9 $mW/cm^2$ in the presence of unusually high concentrations of AMT ($2.8 \times 10^{-4}$ M = 82 µg/ml) and HMT ($2.8 \times 10^{-4}$ M = 72 µg/ml). With 20 minutes of irradiation there was only a 5-fold reduction of infectivity in the case of HMT, whereas there was more than a $10^4$-fold reduction with 15 minutes irradiation with AMT. [K. Nakashima & A. Shatkin, "Photochemical cross-linking of reovirus genome RNA in situ and inactivation of viral transcriptase", J. Biological Chemistry 253: 8680-8682 (1978).]

Nakashima et al. later demonstrated a psoralen photochemical inactivation of vesicular stomatitis virus and influenza virus (both single-stranded RNA viruses), monitored by loss of viral RNA polymerase activity (which is dependent upon intact viral RNA). For both viruses, a 100-fold reduction resulted from approximately 5 minutes irradiation at 9 $mW/cm^2$ in the presence of 8.2 µg/ml AMT. Under the same conditions, HMT was found to be about 10-fold less effective in the photoinactivation of influenza virus. [K. Nakashima, P. K. Chanda, V. Deutsch, A. K. Banerjee & A. Shatkin, "Inactivation of influenza and vesicular stomatitis virion RNA polymerase activites by photoreaction with 4'-substituted psoralens", J. Virol. 32: 838-844 (1979).]

Redfield et al. demonstrated the photoinactivation of cell-free and intracellular influenza and herpes simplex viruses by irradiation at 1.2

$mW/cm^2$ of long-wave ultraviolet light in the presence of various concentrations of AMT and HMT. Herpes virus was photoinactivated $10^4$-fold in 0.9 minutes with 10 µg/ml of either HMT or AMT. At lower concentrations of the psoralen derivatives, AMT was always more effective than an equal amount of HMT. At all psoralen concentrations, influenza virus was more rapidly inactivated with AMT than with equivalent amounts of HMT. In the presence of 10 µg/ml of AMT, cell-free influenza virus was inactivated $10^6$-fold in 1 minute. Intracellular influenza virus required 4 minutes for a $10^7$-fold inactivation under these conditions. [D. C. Redfield, D. D. Richman, M. N. Oxman & L. H. Kronenberg, "Psoralen inactivation of influenza and herpes simplex virus and of virus-infected cells", Infection and Immunity 32: 1216-1226 (1981).]

Swanstrom et al. demonstrated the photochemical inactivation of Rous sarcoma virus with HMT, carried out using BLB fluoroescent ultraviolet lamps in a configuration which, in studies in other laboratories, provided approximately 2 $mW/cm^2$ of long-wave ultraviolet light. (The exact intensity was not determined in this study.) Thirty minutes of irradiation resulted in only a 10-fold reduction in infectivity in the presence of 2.5 µg/ml HMT. A $10^4$-fold inactivation required 30 minutes of irradiation with 25 µg/ml of HMT. [R. Swanstrom, L. M. Hallick, J. Jackson, J. E. Hearst & J. M. Bishop, "Interaction of psoralen derivatives with the RNA genome of Rous sarcoma virus", Virology 113: 613-622 (1981).]

Leick et al. disclose the photoinactivation (using a high intensity of 100 $mW/cm^2$ of long-wave ultraviolet light) of the translational activity (RNA-directed synthesis of polypeptides) of tobacco mosaic virus (TMV), carried out with various psoralen

derivatives. The effects of AMT and HMT were indistinguishable and were slightly greater than the effects of TMP. [V. Leick and P. E. Nielsen, "Photochemical effect of psoralens on TMV-RNA messenger activity in in vitro protein synthesis", Photochem. and Photobiol. 12: 285-290 (1981).]

Talib et al. disclose the inactivation (i.e., elimination of gene expression) of vesicular stomatitis virus, using AMT with 9 $mW/cm^2$ of long-wave ultraviolet light. It was concluded that vesicular stomatitis virus is very unusual in that AMT could photoreact only with a single base (cytosine) of the viral RNA, but that this was sufficient for inactivation. [S. Talib and A. K. Banerjee, "Covalent attachment of psoralen to a single site on vesicular stomatitis genome RNA blocks expression of viral genes", Virology 118: 430-438 (1981).]

Hanson et al. disclose the photochemical inactivation of several single-stranded RNA viruses by 10 µg/ml AMT at 40 $mW/cm^2$ of long-wave ultraviolet light. Influenza virus was found to be exceptionally sensitive, undergoing a $10^4$-fold inactivation in 15 seconds. Measles and parainfluenza viruses required 10-20 times more irradiation under the same conditions for an equivalent inactivation. Venezuelan equine encephalitis virus was also found to be very sensitive, with kinetics of inactivation only slightly slower than that of influenza virus. [C. V. Hanson, "Inactivation of viruses for use as vaccines and immunodiagnostic reagents", in: Medical Virology II (Proceedings of the 1982 International Symposium on Medical Virology), edited by L. M. de la Maza and E. M. Peterson, Elsevier Biomedical, NY, 1983, pp. 45-79.]

The first published report of the existence of HTLV III in patients with acquired immune deficiency

syndrome (AIDS) was by Barre-Sinoussi et al. in Science, Volume 220, p. 868 (1983).

Soon after, Gallo and co-workers at the United States National Institutes of Health published an article describing HTLV III. [Popovic et al., Science, Volume 224, p. 497 (1984)]

Recipients of blood transfusions and blood products, e.g., Factor VIII, are among those in the high risk group for contacting AIDS. Consequently, there is a need for serodiagnostic methods for HTLV III and/or anti-HTLV III antibodies, which methods can be used to screen blood and blood products. A contemplated method comprises a solid phase immunoassay in which purified HTLV III viral antigens are immobilized onto a solid phase. Sera from blood donors are contacted with the solid phase having HTLV III immobilized thereon. Anti-HTLV III antibodies in the sera will bind to the viral antigens on the solid phase. After suitable incubation and washing, the solid phase having immune complex thereon is contacted with a labeled anti-human antibody.

The worldwide testing of blood is anticipated to exceed 100 million tests per year. The commercial production of serodiagnostic test kits requires the production of purified HTLV III. AIDS is known to have a 40-60% mortality rate. To protect not only workers engaged in the production and purification of large volumes of HTLV III containing fluid (greater than 200 liters per week) used in the manufacture of test kits, but also clinicians using the kits in laboratories, as well as to prevent contamination of the general population during commercial shipment of the kits, the use of inactivated HTLV III is required. However the inactivated virus must still be able to react with anti-HTLV III antibodies.

Although various inactivation methods are known, e.g., detergent disruption of virion morphology, heat inactivation of viral enzymes and disruption of viral capsid by dehydration, none specifically inactivates HTLV III nucleic acid. Such nucleic acid inactivation would add an increased margin of safety to inactivated HTLV III products. In addition, it would be desirable to inactivate HTLV III after propagation, but prior to extensive purification, thereby minimizing the risk of accidental infection of personnel engaged in the purification of HTLV III. The use of detergents, heat, dehydrating agents, etc. prior to the purification of HTLV III from tissue culture host cells must be avoided since purification methods require intact virion morphology (substantially unaltered nucleocapsid, capsid and viral envelope). Consequently, there is a need for a method for the rapid inactivation of HTLV III at the nucleic acid level which will not substantially alter the antigenicity or morphology of the virus.

## DISCLOSURE OF THE INVENTION

This need is met by the present invention which is a method for the rapid inactivation of Human Tumor Leukemia Virus III without substantial loss of antigenicity, comprising contacting the virus with a compound of the formula:

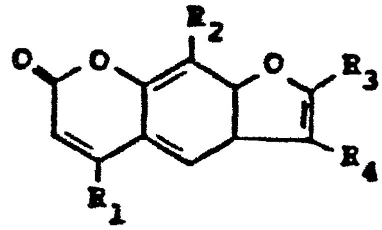

wherein

$R_1$, $R_2$ and $R_3$ are either all H or all $CH_3$; and

$R_4$ is halogenated lower alkyl, hydroxy lower alkyl, lower alkoxy, lower alkyl or primary amino lower alkyl;

and simultaneously or sequentially exposing the resulting mixture to light having a wavelength in the range of 320 to 380 nm.

DESCRIPTION OF THE INVENTION

The literature cited above discloses the ability of psoralen and various derivates thereof to inactivate viruses without altering antigenicity. However, the rates at which these inactivators work vary greatly, depending on the particular psoralen derivative used and on the virus to be inactivated. The literature cited above shows that, in general, the so-called retroviruses, i.e., those containing a substantially single-stranded RNA and reverse transcriptase, are inactivated more slowly than other single stranded RNA viruses, e.g., influenza virus, vesicular stomatitis virus and western equine encephalitis virus.

The rates at which most retroviruses are inactivated, e.g., typically fifteen to thirty minutes, are too slow for the commercial-scale production of inactivated HTLV III, i.e., one to two hundred liters of tissue culture fluid per week to be inactivated.

It has been discovered that HTLV III, a typical retrovirus, can be inactivated using psoralen and selected derivatives thereof in the presence of long wave ultraviolet light at a rate five to ten times faster than the rate at which the typical retrovirus is inactivated. Although psoralen-specific inactivation kinetics similar to those of the present invention have been reported for influenza and herpes simplex, the close morphological similarity between HTLV III and other retroviruses, notably Rous Sarcoma virus, suggests that the inactivation kinetics of HTLV

III would be considerably slower than those actually discovered in the course of developing the present invention.

Psoralen and its derivatives are described in U.S. 4,124,598 issued to Hearst et al. and are available from commercial sources. The HMT and AMT psoralen derivatives are preferred over TMP because they have been shown to provide slightly faster inactivation kinetics. Typical final concentrations are from 0.5 to 50 µg/ml. HTLV III is generally present in crude, unconcentrated tissue culture fluid at concentration ranging from $1 \times 10^5$ virions/mL to $1x10^{13}$ virions/mL and can be inactivated conveniently using the method of the present invention. Either simultaneously or sequentially, the psoralen treated HTLV III is irradiated with long wave ultraviolet light in the range of 320 to 380 nm, preferably 350 to 366 nm, at an incident intensity of 1 $mW/cm^2$ to 200 $mW/cm^2$. The duration of the irradiation is determined empirically using inactivation curves which show surviving virus as a function of treatment time. Inactivation can be performed at any convenient temperature, although temperatures in the range of 0° to 50°C are preferred, 4° to 10°C being most preferred. Because the ultraviolet radiation can heat the reaction mixture, thereby degrading viral antigens, lower temperatures are preferred. A temperature of 4°C is most preferred.

The present invention has its greatest utility in the preparation of commercial quantities (large volumes) of inactivated HTLV III. The preferred commercial method is in the nature of a semi-continuous batch process. Typically, tissue culture fluid containing virus is placed in a vessel with psoralen or a suitable derivative thereof (e.g., AMT, HMT, TMP). The resulting mixture is then made to

flow through optically transparent tubing, a portion of which is located in an ultraviolet irradiation zone. The path length of the tubing within the zone and the flow rate through the tubing can be adjusted to provide for the optimum irradiation exposure. The use of tubing in the shape of a coil permits optimization of irradiation exposure by adjustment of the coil pitch. Additionally, a light source can be placed inside the coil, as well as outside. Preferred optically transparent, i.e., long wave U.V. transparent, tubing includes borosilicate glass and plastics such as polystyrene, polypropylene and Mylar® (E. I. du Pont de Nemours and Co.). The coiled tubing can be placed in a container in which water at a controlled temperature is circulating. The water acts to prevent the ultraviolet light from heating the virus. The use of a cobalt salt in the water acts to enhance heat dissipation and also acts to transmit preferentially ultraviolet light in the preferred range. To maximize biological safety, it is preferred to place the entire apparatus under a biosafety hood.

The following example illustrates the unexpected rapidity with which the method provided by the present invention is able to inactivate HTLV III.

## Example

Initial experiments using psoralen as a method of inactivating HTLV-III virus were designed to answer four fundamental questions which were (1) ability of psoralen to inactivate HTLV-III; (2) most effective concentration of psoralen; (3) most effective light intensity (at approximately 360 nm) measured in $mW/cm^2$; and (4) treatment time [duration of sample (psoralen-HTLV-III solution) irradiation]. The previously published work of Swanstrom et al. (1981) was used as a guide for choosing the parameters of

psoralen concentration: light intensity and time of irradiation. This was the only known, published description of psoralen inactivation of a retrovirus, namely, Rous sarcoma virus (RSV). In addition, this was the only publication which described inactivation kinetics (change in viral infectivity versus time) for psoralen inactivation of a retrovirus or RNA tumor virus. Swanstrom et al. showed that approximately $1 \times 10^5$ FFU/ml (fluorescent focus units/mL) of RSV was inactivated following a 30 min U.V. light exposure (approximately 2 $mW/cm^2$) at a psoralen concentration of 25 µg/mL. Based on these data an experiment was designed to answer the questions of HTLV-III sensitivity to psoralen, effective concentration of psoralen, effective light intensity and range of exposure times. Choice of exposure times was very important for several reasons. First, infectivity assays for HTLV-III are very time consuming and labor intensive, and each assay requires up to 30 days to reach an end point. HTLV-III infectivity assays were performed by adding aliquots of serially diluted, treated virus suspensions to uninfected host cells (H9 cells obtained from R. C. Gallo) and at various times e.g., 14, 21, 28 and 35 days removing cells at each dilution and analyzing for the presence of HTLV-III antigens on the cell surface using an indirect immunofluorescent assay. HTLV-III-infected H9 cells showed a characteristic greenish fluorescence on their periphery using fluorescent microscopy. The end point chosen was the last dilution of HTLV-III showing virus specific fluorescent cell staining. The virus titer or number of infectious virus particles originally present in the undiluted samples was calculated using the reciprocal of the end point. Second, once the relative sensitivity of HTLV-III to psoralen had been

established, additional experiments were necessary using numerous treatment times from zero to that time which gave 100% inactivation in order to establish inactivation kinetics curves or kill curves. Once the kill curve (slope) had been established, one can extend the treatment time beyond that which is known to give 100% inactivation and thereby establish confidence of inactivation or degree of overkill. Since HTLV-III infection in humans may have 100% mortality, the objective using psoralen was to treat with U.V. light long enough to insure that the probability of having one infectious HTLV-III virion was less than $1 \times 10^6$ to $1 \times 10^{10}$ in one milliliter. Third, psoralen inactivation of HTLV-III in large volumes of clarified tissue culture fluid (50-100 liters/batch) was investigated. If this could be accomplished, an enhanced margin of safety to manufacturing personnel engaged in HTLV-III purification could be provided. In addition, psoralen inactivation in combination with other methods used to prepare HTLV-III specific antigens e.g., detergent solubilization, alcohol treatment and drying, would provide absolute assurance of HTLV-III inactivation or absence of infectivity.

Experiments were performed using a starting HTLV-III titer of $1 \times 10^5$FFU/mL. Aliquots of virus were treated as follows: 0, 10, or 20 µg/mL psoralen, each at 5 mW/cm$^2$; 0, 10, and 20µg/mL psoralen, each at 25 mW/cm$^2$; and 0, 10, and 20 µg/mL psoralen, no irradiation. Times for low U.V. exposure ( 5 mW/cm$^2$) for each sample were 0, 15. 30, 45 and 90 mins and for high U.V. exposure (25 mW/cm$^2$) were 0, 1, 2.5, 5, 10, 15, and 30 min. Based on the previous data of Swanstrom et al. (1981) these experimental conditions were predicted to produce varying degrees of HTLV-III

inactivation. Surprisingly, no infectious HTLV-III was recovered from any sample which received any combination of psoralen and irradiation. HTLV-III titers in samples receiving only psoralen and no irradiation, only irradiation and no psoralen and neither psoralen nor irradiation were identical i.e., $1 \times 10^5$ FFU/mL. These data indicated that inactivation of HTLV-III using psoralen was unexpectedly rapid compared to the published psoralen inactivation of Rous sarcoma virus and the unpublished results of Hanson. Additional experiments were designed which were essentially identical to those described above with the primary changes as follows: low U.V. ($5mW/cm^2$) samples were irradiated for 0, 0.5, 1, 2, 4, 8 and 16 minutes and high U.V. ($25mW/cm^2$) samples were irradiated for 0. 10, 20, 40, 90, 18, and 360 seconds. (Purified, high titer HTLV-III ($1 \times 10^9$ FFU/mL) was employed in order to increase the chance of recovering infectious virus from treated samples.) Figure 1 summarizes the results of these experiments for low U.V.-treated HTLV-III samples in the presence of 10µg/mL psoralen. Figure 2 summarizes the results for high U.V.-treated HTLV-III samples in the presence of 10µg/mL psoralen. In summary, the data indicate that $1 \times 10^9$ FFU HTLV-III can be inactivated using psoralen (10µg/mL) and high U.V. ($25mW/cm^2$) in 90 seconds and at low U.V. ($5mW/cm^2$) in 3 min. Assuming that HTLV-III is a typical retrovirus (all known information suggests this to be the case), the psoralen inactivation kinetics are surprising and unexpected compared with what one would expect based on the published psoralen inactivation of Rous sarcoma virus. The rapidity of the inactivation makes the method of the present invention suitable for inactivation of large volumes of clarified tissue culture fluid using a semi-continuous batch process as described earlier.

CLAIMS

1. A method for the rapid inactivation of Human Tumor Leukemia Virus III without substantial loss of antigenicity which comprises contacting the virus with a compound of the formula:

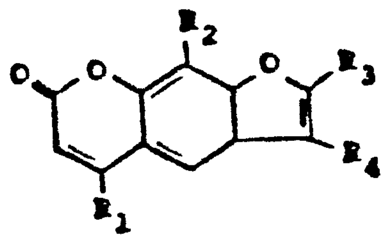

wherein $R_1$, $R_2$ and $R_3$ are either all H or all $CH_3$ and $R_4$ is halogenated lower alkyl, hydroxy lower alkyl, lower alkoxy, lower alkyl or primary amino lower alkyl, and simultaneously or sequentially exposing the resulting mixture to light having a wavelength of 320 to 380 nm.

2. A method according to claim 1 wherein $R_1$, $R_2$ and $R_3$ are all $CH_3$.

3. A method according to claim 1 or 2 wherein $R_4$ is primary amino lower alkyl.

4. A method according to claim 3 wherein $R_4$ is amino-methyl.

5. A method according to any one of the preceding claims wherein the temperature is $0^{o}$ to $50^{o}C$.

6. A method according to claim 5 wherein the temperature is $4^{o}C$.

7. A method according to any one of the preceding claims wherein the light has a wavelength of 350-366 nm.

8. A method according to any one of the preceding claims wherein the light is of an intensity of 1 $mW/cm^2$ to 200 $mW/cm^2$.

9. A method according to any one of the preceding claims wherein the compound is used at a final concentration 0.5 to 50 $\mu g/mL$.

10. A method according to any one of the preceding claims wherein $R_4$ contains 1 to 4 carbon atoms.

FIG. I

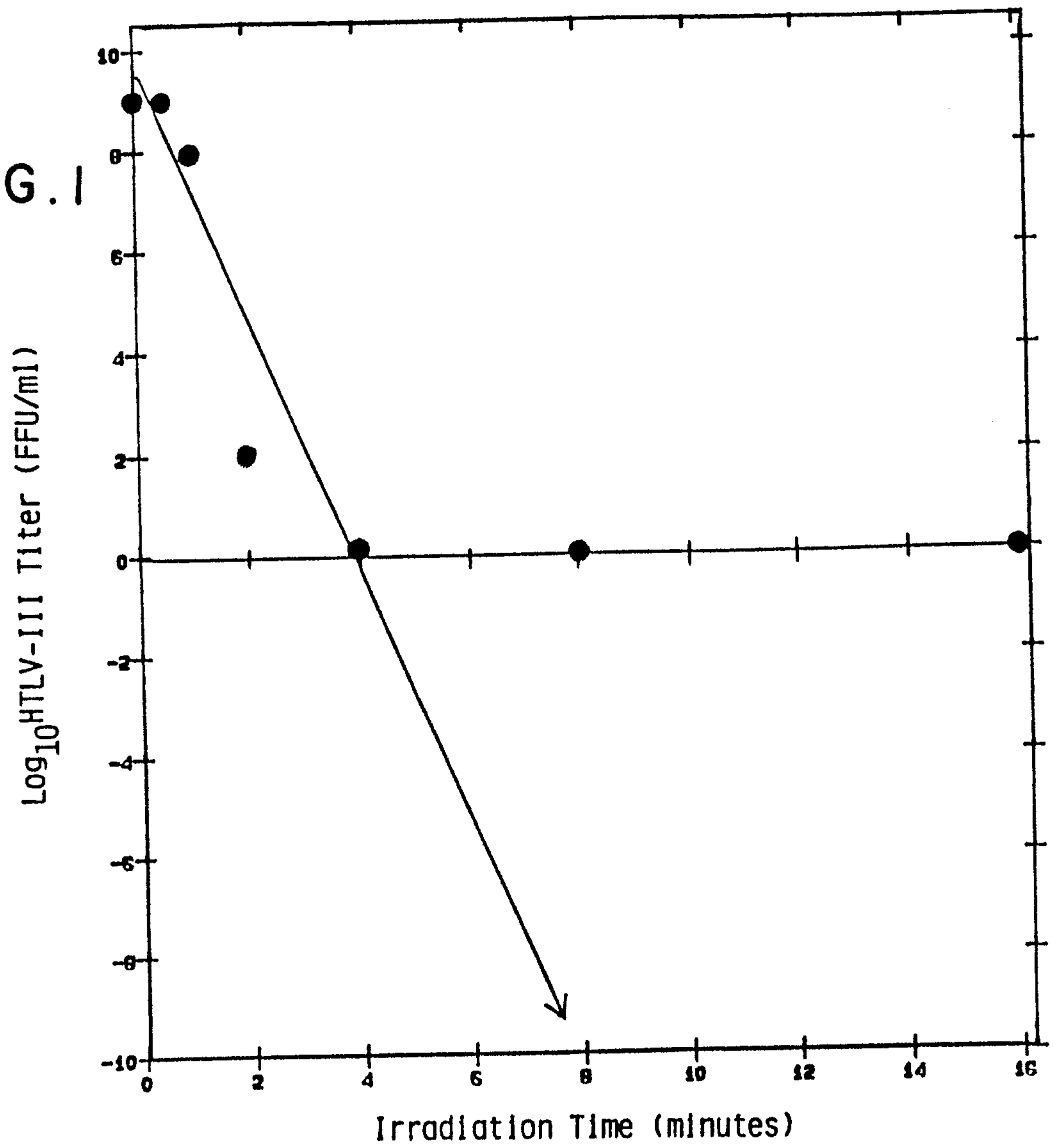


Psoralen[1]/U.V. (Low U.V.)[2]

Inactiovation Kinetics
for
HTLV-III

[1] (Psoralen) = 10µg/mL

[2] U.V.$_{360nm}$ = 5mW/cm$^2$

3 No infectious virus recovered at T=4, 8 and 16 minutes; slope extrapolated below 0 on Y axis.

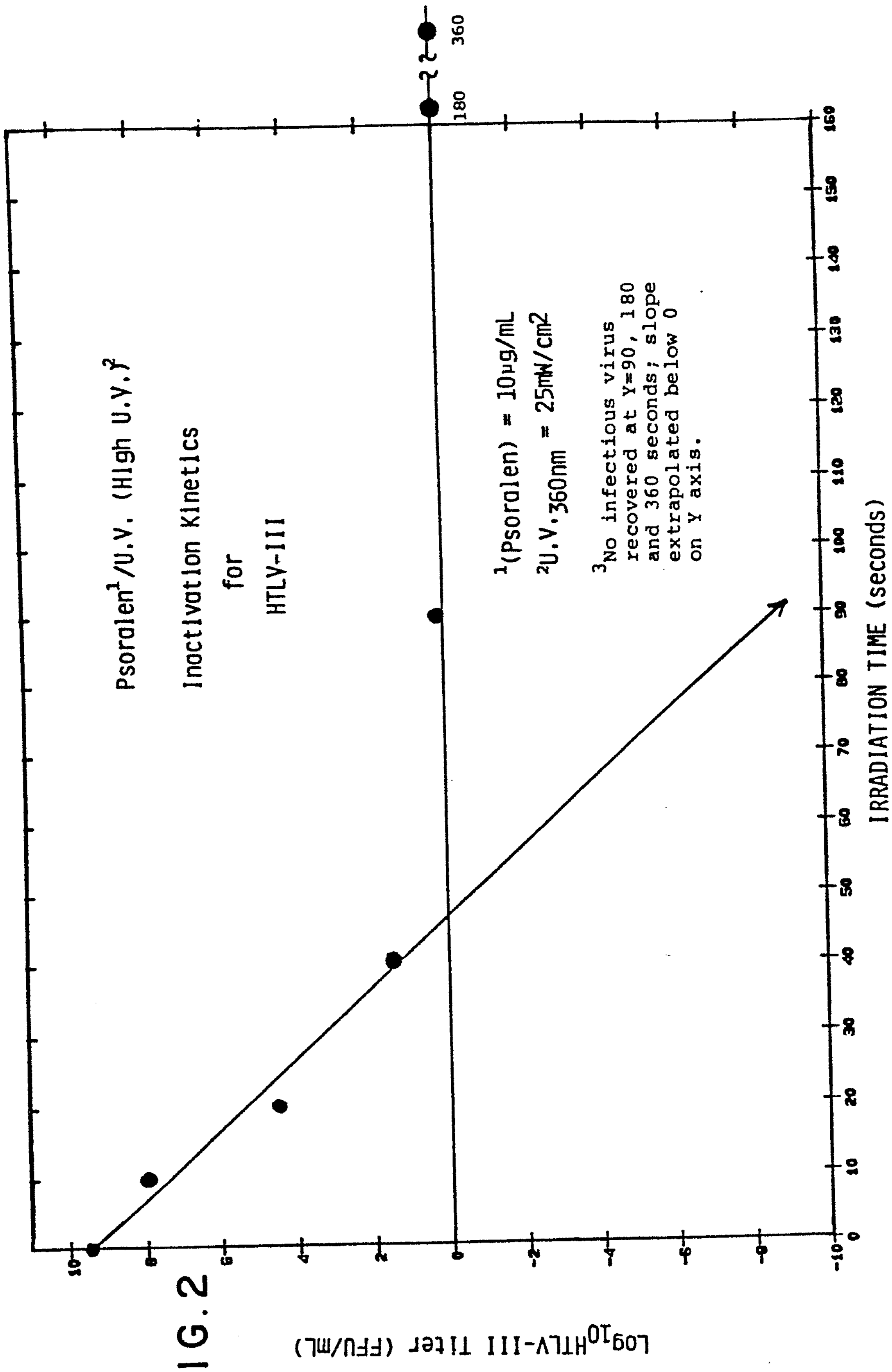
Psoralen¹/U.V. (High U.V.)²
Inactivation Kinetics
for
HTLV-III
¹(Psoralen) = 10µg/mL
²U.V.360nm = 25mW/cm²
³No infectious virus recovered at Y=90, 180 and 360 seconds; slope extrapolated below 0 on Y axis.
IRRADIATION TIME (seconds)
Log10HTLV-III Titer (FFU/ML)
0
10
20
30
40
50
60
70
80
90
100
110
120
130
140
150
160
180
360
10
8
6
4
2
0
-2
-4
-6
-8
-10

FIG.2